# FASCICULE DE BREVET EUROPEEN

(11) **EP 1 208 873 B1**
(45) Date de publication et mention de la délivrance du brevet: **28.09.2005**
(21) Numéro de dépôt: 01403023.3
(22) Date de dépôt: 26.11.2001
(51) Int. Cl.: A61N 1/362, A61N 1/39

(54) **Dispositif médical actif du type défibrillateur/cardioverteur implantable à discrimination perfectionnée des fibrillations auriculaires**
Aktive, medizinische Vorrichtung des Typs implantierbarer Defibrillator/Kardioverter mit verbesserter Unterscheidung von Vorhoffibrillation
Active medical device of the implantable defibrillator/cardioverter type with improved atrial fibrillation discrimination

(30) Priorité: 27.11.2000 FR 0015293
(43) Date de publication de la demande: 29.05.2002
(73) Titulaire: ELA MEDICAL, 92541 Montrouge (FR)
(72) Inventeur: Henry, Christine, 75014 Paris (FR); Limousin, Marcel, 75014 Paris (FR)
(74) Mandataire: Dupuis-Latour, Dominique

(56) Documents cités:
- EP-A- 0 879 621
- US-A- 5 107 850
- US-A- 5 350 406
- US-A- 5 868 793

## Description

La présente invention concerne les dispositifs médicaux implantables actifs (au sens de la directive 90/385/CEE du 20 juin 1990 du Conseil des communautés européennes), et plus particulièrement la famille des appareils chargés de délivrer au coeur des impulsions électriques de haute énergie (c'est-à-dire dépassant notablement l'énergie fournie pour la simple stimulation) en vue de mettre fin à une tachyarythmie. Ces modes de thérapie incluent également un mode de stimulation programmée à haute fréquence ou "ATP" (*AntiTachycardia Pacing*).

Ces dispositifs sont communément appelés "défibrillateurs implantables" ou "appareils de cardioversion" (étant entendu que l'invention couvre aussi bien les défibrillateurs/cardioverteurs/stimulateurs implantables que les défibrillateurs/stimulateurs implantables).

Ces dispositifs comprennent un générateur d'impulsions chargé de surveiller l'activité cardiaque et de générer des impulsions de stimulation ou de haute énergie quand le coeur présente une arythmie ventriculaire susceptible d'être traitée (quand cette énergie est comprise entre 0,1 et 10 J environ, on désigne cette thérapie sous le nom de "cardioversion" et le choc électrique est appelé "choc de cardioversion". Quand cette énergie est supérieure à 10 J environ, le choc électrique est alors appelé "choc de défibrillation").

Ce choc doit être délivré lorsque l'on détecte une tachycardie ventriculaire (TV), mais à condition qu'il s'agisse bien d'une véritable TV, et non d'une tachycardie supraventriculaire (TSV) ; en effet, dans ce dernier cas, la tachycardie est d'origine auriculaire et le choc qui serait délivré serait sans effet puisque l'électrode de défibrillation ou, le cas échéant, de stimulation, ne se trouve pas dans cette région.

En réalité, ces situations recouvrent diverses formes de trouble du rythme cardiaque : lorsque l'on se trouve en présence d'un rythme cardiaque rapide anormal (tachyarythmie), celui-ci peut avoir pour cause une fibrillation ventriculaire (FV), une tachycardie ventriculaire (TV), une tachycardie sinusale (TS) ou une tachycardie supraventriculaire (TSV). La tachycardie supraventriculaire recouvre elle-même la tachycardie auriculaire, le flutter auriculaire et la fibrillation auriculaire (FA).

Ces troubles peuvent d'ailleurs s'ajouter, et l'on parle alors de "bitachycardie", notamment en présence d'une fibrillation auriculaire combinée à une tachycardie ventriculaire.

Le diagnostic des tachycardies peut être opéré, de manière en elle-même connue, à partir de critères tels que la fréquence ventriculaire, la stabilité des intervalles ventriculaires (intervalles RR), l'analyse de l'association auriculo-ventriculaire (révélée par la stabilité de l'intervalle PR) et le mode de démarrage des tachycardies (présence d'une accélération brusque et cavité d'origine, ventriculaire ou auriculaire).

On pourra notamment se référer au EP-A-0 626 182 (Ela Médical), qui décrit un algorithme de détection et de classification des tachyarythmies mis en oeuvre en particulier dans le modèle *Defender 9001* d'Ela Médical.

Les EP-A-0 838 235 et EP-A-0 813 888 (Ela Médical) décrivent divers perfectionnements à cet algorithme, permettant d'améliorer encore la discrimination entre TV et TSV, notamment pour éviter des faux diagnostics positifs (indication d'une TV alors qu'il s'agissait d'une TSV) ou négatifs (indication d'une TSV alors qu'il s'agissait d'une TV).

Le point de départ de la présente invention réside dans l'observation de certaines situations cliniques où l'algorithme continue néanmoins à être leurré, conduisant à un faux diagnostic de TV et donc à un risque d'application d'une thérapie inappropriée.

Le but de l'invention est de remédier à cette situation, en réduisant encore les risques associés à ces situations.

L'analyse clinique a montré le besoin de traiter spécifiquement, dans l'algorithme de détection des tachycardies, le cas où une fibrillation auriculaire (FA) est présente en même temps qu'une réponse ventriculaire rapide et stable.

En effet, l'arbre de décision de l'algorithme connu est tel que, si le rythme ventriculaire est à la fois rapide (c'est-à-dire supérieur à la fréquence de seuil de détection d'une TV) et stable, et que l'intervalle PR n'est pas stable, c'est-à-dire en l'absence d'association auriculo-ventriculaire, alors le dispositif considère que le patient est en TV. Or, dans un type d'arythmie avec FA et réponse ventriculaire rapide, la fréquence élevée du rythme accroît la stabilité, et la FA induit une instabilité de l'intervalle PR. Mais une FA, qui est une forme de TSV, n'est pas justiciable d'une thérapie de choc ventriculaire, à la différence d'une véritable TV.

Il convient donc de perfectionner l'algorithme de manière à apporter une discrimination supplémentaire permettant de reconnaître et traiter spécifiquement cette situation.

L'invention propose à cet effet un dispositif du type décrit par le EP-A-0 626 182 précité, c'est-à-dire comprenant : des moyens de délivrance d'une thérapie de défibrillation et/ou de cardioversion et/ou de stimulation antitachycardique ventriculaire et/ou auriculaire ; des moyens de recueil de l'activité ventriculaire et auriculaire ; des moyens pour suspecter et confirmer la présence d'épisodes de tachycardie dans l'activité ainsi recueillie, ces moyens comprenant des moyens pour analyser la stabilité des intervalles RR et la stabilité des intervalles PR associés, et des moyens discriminateurs mis en oeuvre en cas de détection d'intervalles RR stables et d'intervalles PR instables, ces moyens étant aptes à discriminer entre fibrillation auriculaire à réponse ventriculaire rapide et tachycardie ventriculaire, et à commander une thérapie différenciée selon l'un ou l'autre cas.

Selon l'invention, les moyens discriminateurs comportent des moyens du groupe comprenant:
- des moyens de mesure de l'amplitude des ondes P et d'analyse de la stabilité de ces amplitudes, pour déterminer la présence d'une fibrillation auriculaire en cas d'instabilité supérieure à un seuil donné, et/ou
- des moyens d'inhibition temporaire des moyens de recueil de l'activité ventriculaire, et de détection de capture auriculaire, pour déterminer la présence d'une fibrillation auriculaire en cas d'absence de capture auriculaire, et/ou
- des moyens d'évaluation du délai de conduction entre oreillette droite et oreillette gauche, et d'analyse de la stabilité de ce délai, pour déterminer la présence d'une fibrillation auriculaire en cas d'instabilité supérieure à un seuil donné.

Très avantageusement, le dispositif comporte également des moyens de détection de bitachycardie et de commande d'une thérapie adaptée, ces moyens étant mis en oeuvre lorsque les moyens discriminateurs détectent la présence d'une fibrillation auriculaire. Il peut s'agir :
- de moyens d'analyse de la durée des intervalles RR, pour déterminer la présence d'une bitachycardie en cas de détection d'au moins un cycle de durée supérieure à un seuil donné au cours d'une série de cycles cardiaques successifs, et/ou
- de moyens d'application d'une thérapie de choc auriculaire à basse énergie et d'évaluation de l'efficacité de cette thérapie, pour déterminer la présence d'une bitachycardie en cas de persistance de la fibrillation auriculaire après thérapie, et/ou
- de moyens de temporisation, pour déterminer la présence d'une bitachycardie en cas de persistance de la fibrillation auriculaire en fin de temporisation.

On va maintenant décrire plus en détails différents modes de mise en oeuvre des enseignements de l'invention.

Le dispositif de l'invention utilise un algorithme de détection et de classification des tachyarythmies d'un défibrillateur tel que le modèle *Defender 9001* d'Ela Médical, algorithme que l'on a perfectionné de la manière que l'on va indiquer plus bas.

La première étape de l'analyse consiste à isoler le cas particulier dans lequel :
- le rythme ventriculaire est rapide, c'est-à-dire supérieur à la fréquence seuil de diagnostic de TV,
- le rythme ventriculaire est stable, critère évalué à partir des intervalles RR, et
- il n'y a pas d'association auriculo-ventriculaire, critère évalué à partir de la stabilité des intervalles PR.

Plus précisément, l'appareil décrit dans le EP-A-0 626 182 précité définit qu'il y a stabilité des intervalles RR lorsque le pic d'autocorrélation, divisé par le total d'autocorrélation, excède un rapport déterminé (le pic d'autocorrélation est le nombre maximal d'intervalles récents dans le ventricule qui satisfont à un critère de stabilité prédéterminé).

Il définit qu'il y a stabilité de conduction lorsque la valeur du pic d'intercorrélation, divisée soit par la valeur du pic d'autocorrélation, soit, en variante, par le total d'intercorrélation, excède un rapport déterminé (le pic d'intercorrélation est le nombre maximal d'intervalles de conduction en provenance de l'oreillette qui satisfont un critère de stabilité prédéterminé). En d'autres termes, dans le premier cas précité on compare la stabilité de la conduction entre les deux chambres à celles des intervalles dans le ventricule, tandis que dans le second cas on exprime la stabilité de la conduction entre les deux chambres en fonction de la totalité des conductions présumées.

Si l'on se trouve ainsi dans le cas d'un rythme ventriculaire rapide et stable et d'une conduction instable, on procède alors, de façon caractéristique de l'invention, à une analyse complémentaire de manière à déterminer si l'on se trouve en présence d'une TV ou d'une FA à réponse rapide.

Cette analyse peut être opérée de plusieurs manières différentes.

Une *première technique* consiste à mesurer l'amplitude des ondes P et à évaluer les variations cycle à cycle de celles-ci.

Si cette variabilité est élevée, alors l'algorithme considère qu'il y a présence d'une FA.

Le critère de variabilité de l'amplitude peut être par exemple considéré comme vérifié lorsque l'on mesure une variation du niveau d'amplitude supérieure à 20 % sur au moins deux cycles parmi quatre cycles successifs.

Une *deuxième technique* consiste à analyser la fréquence auriculaire, et à considérer que l'on est en présence d'une FA si la fréquence auriculaire est supérieure à la fréquence ventriculaire.

Plus précisément, pour permettre cette évaluation, le dispositif est commuté de mode double chambre (DDD) en mode simple chambre (AAI) pour améliorer l'écoute auriculaire en évitant que celle-ci ne soit perturbée par la détection du ventricule. En effet, l'analyse de la FA peut être perturbée par la présence des périodes réfractaires auriculaires absolues, qui limitent la détection de certaines ondes P.

Une fois effectué le diagnostic du rythme ventriculaire rapide et stable (qui donne donc une valeur de référence de fréquence ventriculaire), l'appareil recueille et analyse la fréquence auriculaire, par exemple sur une durée de quatre à cinq secondes, dans un mode de détection auriculaire uniquement (mode AAI). Une fréquence auriculaire supérieure à la fréquence ventriculaire de référence révèle alors la présence d'une FA.

Cette technique de détection des FA est plus particulièrement adaptée au cas où le patient est appareillé avec une sonde de détection auriculaire à dipôle court, qui réduit notablement le risque de détection des dépolarisations ventriculaires.

Une *troisième technique* consiste à effectuer un test de capture auriculaire après stimulation, c'est-à-dire du seuil de tension de l'amplitude de l'impulsion de stimulation nécessaire pour provoquer une dépolarisation de la cavité à laquelle est appliquée cette impulsion. La détection du seuil de capture ou "seuil d'entraînement" est par exemple opérée de la manière décrite dans le EP-A-0 552 357 (Ela Médical).

Le dispositif (ici encore de préférence commuté temporairement en mode AAI pour améliorer l'écoute auriculaire) délivre une stimulation auriculaire (maximum : 5 V et 1 ms) avec un couplage supérieur à 200 ms par rapport à la dernière détection, puis mesure le potentiel évoqué. Si le dispositif de mesure du seuil d'entraînement confirme l'existence d'une capture auriculaire, alors l'appareil considère qu'il est en présence d'une véritable TV ; dans le cas contraire il considère qu'il s'agit d'une FA, car une stimulation de l'oreillette en présence d'une FA ne produit qu'exceptionnellement une capture auriculaire.

Une *quatrième technique* repose sur l'une des caractéristiques de la FA, qui est la variabilité de la dépolarisation des oreillettes, caractérisable par un délai de conduction oreillette droite/oreillette gauche très variable.

Cette technique est avantageusement mise en oeuvre avec un dispositif multisite, où le patient est appareillé avec une sonde de détection dans l'oreillette droite et une sonde de détection dans l'oreillette gauche. Avantageusement, ces sondes sont des sondes à dipôle court pour limiter les risques de détection des dépolarisations ventriculaires. Les deux sondes auriculaires sont reliées chacune à un amplificateur de détection distinct, et la mesure de l'intervalle droit/gauche est effectuée sur plusieurs cycles consécutifs. De préférence, le dispositif est commuté temporairement en mode AAI pour améliorer l'écoute auriculaire. Si les variations des intervalles mesurés s'étendent sur une plage large, par exemple de plus de 50 ms, alors le dispositif considère qu'il est en présence d'une FA.

Un perfectionnement aux techniques décrites ci-dessus consiste, lorsque l'appareil détermine la présence d'une FA, à opérer une discrimination supplémentaire pour déterminer s'il s'agit d'une FA simple ou d'une "bitachycardie", c'est-à-dire de la combinaison d'une FA et d'une TV, ces deux cas étant justiciables de thérapies différentes.

Cette discrimination supplémentaire peut être opérée de plusieurs manières différentes.

Une *première technique* consiste à appliquer au patient une thérapie avec un choc auriculaire basse énergie (choc léger) et à analyser à nouveau le rythme cardiaque. Si le choc a mis fin au trouble, il s'agissait probablement d'une FA simple ; en revanche, l'échec du choc auriculaire peut confirmer la TV, et il est donc nécessaire d'appliquer une thérapie ventriculaire (ATP éventuellement) ou un choc fort.

Un *deuxième technique* consiste, avant toute action, à laisser passer un certain délai et à analyser à nouveau le rythme cardiaque pour voir si le trouble a disparu. En effet, on sait que la FA conduite est un trouble qui agit par salves. Si le trouble a disparu spontanément en fin de temporisation, on peut conclure qu'il s'agit vraisemblablement d'une FA simple. En revanche, si le trouble persiste, on se trouve vraisemblablement en présence d'une TV combinée à la FA, et le dispositif délivre alors la thérapie ventriculaire.

Une *troisième technique* repose sur la constatation du fait que les salves de FA conduite sont entrecoupées d'un ou plusieurs cycles longs. La discrimination des bitachycardies peut ainsi être opérée par recherche d'un cycle long, par exemple recherche d'un cycle de couplage de durée supérieure à la moyenne des quatre dernières durées RR mesurées pendant une période de tachycardie augmentée de 63 ms. Si un tel cycle est trouvé dans les 24 derniers cycles analysés, le dispositif considérera qu'il y a présence d'une FA ; dans le cas contraire, il considérera qu'il y a TV, donc bitachycardie. Le EP-A-0 813 888 (Ela Médical) décrit un tel procédé de recherche d'un cycle long, pour modifier temporairement les paramètres et/ou les critères de classification d'un algorithme d'analyse des tachycardies.

## Revendications

1. Dispositif médical actif du type défibrillateur/cardioverteur implantable, comprenant :
- des moyens de délivrance d'une thérapie de défibrillation et/ou de cardioversion et/ou de stimulation antitachycardique ventriculaire et/ou auriculaire,
- des moyens de recueil de l'activité ventriculaire et auriculaire,
- des moyens pour suspecter et confirmer la présence d'épisodes de tachycardie dans l'activité ainsi recueillie, ces moyens comprenant des moyens pour analyser la stabilité des intervalles RR et la stabilité des intervalles PR associés, et
- des moyens discriminateurs mis en oeuvre en cas de détection d'intervalles RR stables et d'intervalles PR instables, aptes à discriminer entre fibrillation auriculaire à réponse ventriculaire rapide et tachycardie ventriculaire, et à commander une thérapie différenciée selon l'un ou l'autre cas,
dispositif **caractérisé en ce que** lesdits moyens discriminateurs comportent des moyens du groupe comprenant :
a) des moyens de mesure de l'amplitude des ondes P et d'analyse de la stabilité de ces amplitudes, pour déterminer la présence d'une fibrillation auriculaire en cas d'instabilité de ces amplitudes supérieure à un seuil donné ; et/ou
b) des moyens d'inhibition temporaire des moyens de recueil de l'activité ventriculaire, et de détection de capture auriculaire, pour déterminer la présence d'une fibrillation auriculaire en cas d'absence de capture auriculaire ; et/ou
c) des moyens d'évaluation du délai de conduction entre oreillette droite et oreillette gauche, et d'analyse de la stabilité de ce délai, pour déterminer la présence d'une fibrillation auriculaire en cas d'instabilité de ce délai supérieure à un seuil donné.

2. Le dispositif de la revendication 1, comprenant en outre des moyens de détection de bitachycardie et de commande d'une thérapie adaptée, ces moyens étant mis en oeuvre lorsque les moyens discriminateurs détectent la présence d'une fibrillation auriculaire.

3. Le dispositif de la revendication 2, dans lequel les moyens de détection de bitachycardie comportent des moyens d'analyse de la durée des intervalles RR, pour déterminer la présence d'une bitachycardie en cas de détection d'au moins un cycle de durée supérieure à un seuil donné au cours d'une série de cycles cardiaques successifs.

4. Le dispositif de la revendication 2, dans lequel les moyens de détection de bitachycardie comportent des moyens d'application d'une thérapie de choc auriculaire à basse énergie et d'évaluation de l'efficacité de cette thérapie, pour déterminer la présence d'une bitachycardie en cas de persistance de la fibrillation auriculaire après thérapie.

5. Le dispositif de la revendication 2, dans lequel les moyens de détection de bitachycardie comportent des moyens de temporisation, pour déterminer la présence d'une bitachycardie en cas de persistance de la fibrillation auriculaire en fin de temporisation.

## Patentansprüche

1. Aktive medizinische Vorrichtung vom Typ implantierbarer Defibrillator/Kardioverter, umfassend:
- Mittel zur Anwendung einer Defibrillations- und/oder Kardioversionstherapie und/oder zur ventrikulären und/oder Vorhof-bezogenen Antitachykardie-Stimulation,
- Mittel zum Sammeln der ventrikulären und Vorhof-bezogenen Aktivität,
- Mittel zum Vermuten und Bestätigen der Anwesenheit von Tachykardie-Episoden in der so gesammelten Aktivität, wobei diese Mittel Mittel zum Analysieren der Stabilität der RR Intervalle und der zugeordneten PR Intervalle umfassen, und
- Unterscheidungsmittel, die im Fall der Ermittlung stabiler RR Intervalle und instabiler PR Intervalle eingesetzt werden, dazu geeignet, zwischen Vorhof-Fibrillation mit schneller ventrikulärer Antwort und ventrikulärer Tachykardie zu unterscheiden und eine differenzierte Therapie gemäß dem einen oder dem anderen Fall anzuweisen,
wobei die Vorrichtung **dadurch gekennzeichnet ist, dass** die Unterscheidungsmittel Mittel aus der Gruppe umfassen, die umfasst:
a) Mittel zur Messung der Amplitude der P-Wellen und zur Analyse der Stabilität dieser Amplituden, um die Anwesenheit einer Vorhof-Fibrillation im dem Fall zu ermitteln, dass die Instabilität dieser Amplituden größer als eine gegebene Schwelle ist; und/oder
b) Mittel zur zeitweiligen Hemmung der Mittel zum Sammeln der ventrikulären Aktivität und zur Entdeckung des Vorhofreizes, um die Anwesenheit einer Vorhof-Fibrillation im Fall der Abwesenheit eines Vorhofreizes zu ermitteln; und/oder
c) Mittel zur Beurteilung der Verzögerung der Leitung zwischen rechtem Herzvorhof und linkem Herzvorhof und zur Analyse der Stabilität dieser Verzögerung, um die Anwesenheit einer Vorhof-Fibrillation in dem Fall zu ermitteln, dass die Instabilität dieser Verzögerung größer als eine gegebene Schwelle ist.

2. Vorrichtung gemäß Anspruch 1, darüber hinaus umfassend Mittel zur Entdeckung einer Bitachykardie und zur Anweisung einer geeigneten Therapie, wobei diese Mittel eingesetzt werden, wenn die Unterscheidungsmittel die Anwesenheit einer Vorhof-Fibrillation entdecken.

3. Vorrichtung gemäß Anspruch 2, in der die Mittel zur Entdeckung der Bitachykardie Mittel zur Analyse der Dauer der RR Intervalle umfassen, um die Anwesenheit einer Bitachykardie im Fall der Entdeckung mindestens eines Zyklus von größerer Dauer als eine gegebene Schwelle im Lauf einer Serie von aufeinander folgenden Herzzyklen zu ermitteln.

4. Vorrichtung gemäß Anspruch 2, in der die Mittel zur Entdeckung der Bitachykardie Mittel zur Anwendung einer Vorhof-Schock-Therapie mit niedriger Energie umfassen, und zur Beurteilung der Effizienz dieser Therapie, um die Anwesenheit einer Bitachykardie im Fall der Fortdauer der Vorhof-Fibrillation nach der Therapie zu ermitteln.

5. Vorrichtung gemäß Anspruch 2, in der die Mittel zur Entdeckung der Bitachykardie Verzögerungsmittel umfassen, um die Anwesenheit einer Bitachykardie im Fall der Fortdauer der Vorhof-Fibrillation am Ende der Verzögerung zu ermitteln.

## Claims

1. An active medical device of the implantable defibrillator/cardiovertor type, including:
- means for delivering a defibrillation and/or cardioversion therapy and/or for atrial and/or ventricular antitachycardic stimulation,
- means for sensing the ventricular and atrial activity,
- means for suspecting and confirming the presence of episodes of tachycardia in the activity thus sensed, said means including means for analyzing the stability of the RR intervals and the stability of the associated PR intervals, and
- discriminating means operated in the event of a detection of stable RR intervals and unstable PR intervals, for discriminating between atrial fibrillation with fast ventricular response and ventricular fibrillation with ventricular tachycardia, and for controlling a differentiated therapy according to either case,
said device being **characterised in that** said discriminating means include means from the group including:
a) means for measuring the amplitude of P-waves and for analyzing the stability of said amplitudes, for determining the presence of an atrial fibrillation in the event of an instability of said amplitudes greater than a preselected threshold; and/or
b) means for temporarily inhibiting the means for sensing the ventricular activity, and for detecting an atrial capture, for determining the presence of an atrial fibrillation in the event of an absence of atrial capture; and/or
c) means for evaluating the conduction delay between the right atrium and the left atrium, and for analyzing the stability of said delay, for determining the presence of an atrial fibrillation in the event of a instability of said delay greater than a preselected threshold.

2. The device of claim 1, further comprising means for detecting bi-tachycardia and for controlling an adapted therapy, said means being operated when the discriminating means detect the presence of an atrial fibrillation.

3. The device of claim 2, wherein the bi-tachycardia detecting means include means for analyzing the duration of RR intervals, for determining the presence of a bi-tachycardia in the event of a detection of at least one cycle of a duration greater than a given threshold during a series of successive cardiac cycles.

4. The device of claim 2, wherein the bi-tachycardia detecting means include means for applying an atrial shock therapy of low energy and for evaluating the effectiveness of said therapy, for determining the presence of a bi-tachycardia in the event of a persistence of atrial fibrillation after therapy.

5. The device of claim 2, wherein the bi-tachycardia detecting means include delaying means, for determining the presence of a bi-tachycardia in the event of a persistence of atrial fibrillation at the end of the delay.
